# EUROPEAN PATENT SPECIFICATION

(11) **EP 3 329 914 B1**
(45) Date of publication and mention of the grant of the patent: **18.02.2026**
(21) Application number: 16830426.9
(22) Date of filing: 21.07.2016
(51) Int. Cl.: A61K 9/70, A61K 47/02, A61K 47/32, A61K 31/407, A61P 25/18

(54) **METHOD FOR MANUFACTURING ASENAPINE-CONTAINING PATCH**
VERFAHREN ZUR HERSTELLUNG EINES ASENAPINHALTIGEN PFLASTERS
PROCÉDÉ DE FABRICATION D'UN TIMBRE CONTENANT DE L'ASÉNAPINE

(30) Priority: 27.07.2015 JP 2015147516
(43) Date of publication of application: 06.06.2018
(73) Proprietor: Hisamitsu Pharmaceutical Co., Inc., Tosu-shi, Saga 841-0017 (JP)
(72) Inventor: SONOBE, Atsushi, Tsukuba-shi Ibaraki 305-0856 (JP); YASUKOCHI, Takashi, Tsukuba-shi Ibaraki 305-0856 (JP)
(74) Representative: Grünecker Patent- und Rechtsanwälte PartG mbB
(86) International application number: PCT/JP2016/071449
(87) International publication number: WO 2017/018321

(56) References cited:
- EP-A1- 3 329 915
- EP-A1- 3 357 945
- WO-A1-2011/136283
- WO-A1-2013/027052
- WO-A1-2014/017593
- WO-A1-2014/017594
- WO-A1-2014/017595
- AR-A1- 097 544

## Description

### Technical Field

The present invention relates to a method for manufacturing an asenapine-containing patch.

### Background Art

Asenapine is a compound known as a therapeutic agent of central nervous system diseases, in particular, schizophrenia. A patch containing asenapine is described in, for example, Patent Literatures 1 to 4. A patch containing asenapine free base is known to be excellent in the skin permeability of asenapine.
Patent Literature 5 relates to a medical patch containing an adhesive composition containing a salt of a basic drug and a star-shaped acrylic block polymer as an adhesive that exhibits high drug efficacy, that does not cause a decrease in adhesive property of an adhesive base due to an additive, and that causes less skin irritation. The star-shaped acrylic block polymer has a star-shaped structure in which at least three chain polymer portions radiate from a mercapto group situated at the center, wherein (meth)acrylic acid alkyl ester structural units having 7-17 carbon atoms account for 30-99.9% by mass of whole structural units of the star-shaped acrylic block polymer, and at least one of the chain polymer portions has a copolymer structure of polymerizable monomers containing a (meth)acrylic acid alkyl ester having 7-17 carbon atoms and a weakly basic monomer.
Patent Literature 6 describes a patch comprising a backing and an adhesive layer laminated on one side of the backing, wherein the adhesive layer comprises asenapine or a pharmaceutically acceptable salt thereof, an adhesive base and a low molecular weight amine.

Patent literature 7 describes a device for the transdermal delivery of asenapine, which comprises an adhesive matrix layer comprising asenapine in its free base form, an acrylate copolymer, ethyl cellulose, triethylcitrate and hydrochloric acid.

### Citation List

### Patent Literature

Patent Literature 1: WO 2010/127674
Patent Literature 2: WO 2014/017593
Patent Literature 3: WO 2014/017594
Patent Literature 4: WO 2014/017595
Patent Literature 5: EP 3 357 945
Patent Literature 6: EP 3 329 915
Patent Literature 7: WO 2013/027052

### Summary of Invention

### Technical Problem

Meanwhile, when the patch comprises an adhesive layer containing a (meth)acrylic acid ester (co)polymer, use of an asenapine free base in the manufacturing of the patch may lead to easy degradation of asenapine. Therefore, an object of the present invention is to provide a method for manufacturing the patch in which asenapine is not easily degraded.

### Solution to Problem

In processes of investigation of methods in which asenapine is not easily degraded in the manufacturing of the patch, the present inventors have found that degradation of asenapine is reduced when the adhesive layer comprises a metal maleate. They further found that the amounts of degradation products produced of asenapine can be more reduced when an adhesive composition comprising an acid addition salt of asenapine and a desalting agent is used in the manufacturing of the patch, compared to the case in which an adhesive composition comprising an asenapine free base and a metal maleate is used.

Meanwhile, according to the investigation of the present inventors, in the manufacturing of a patch comprising an adhesive layer containing a rubber adhesive, degradation of asenapine was hardly observed in the manufacturing of the patch. The present inventors suppose that degradation of asenapine in the manufacturing of the patch tends to be caused easily by contact with other components in the adhesive composition, heating or exposure to light such as UV rays.

Therefore, the present invention provides a method for manufacturing a patch comprising a backing and an adhesive layer laminated on the backing, comprising: a step of mixing an acid addition salt of asenapine, a (meth)acrylic acid ester (co)polymer and a desalting agent to obtain an adhesive composition, wherein the desalting agent is selected from the group consisting of an alkali metal hydroxide, an alkaline-earth metal hydroxide, and an alkaline-earth metal salt; and a step of shaping the adhesive composition to obtain the adhesive layer, wherein such adhesive layer does not contain a rubber adhesive.

It is preferable that the acid in the acid addition salt of asenapine be selected from the group consisting of hydrochloric acid, hydrobromic acid, hydriodic acid, phosphoric acid, acetic acid, propionic acid, glycolic acid, maleic acid, malonic acid, succinic acid, tartaric acid, citric acid, ascorbic acid, salicylic acid and benzoic acid.

The present invention provides a method for inhibiting the degradation of asenapine in a manufacturing process of a patch comprising an adhesive layer comprising asenapine, comprising: a step of mixing an acid addition salt of asenapine, a (meth)acrylic acid ester (co)polymer and a desalting agent to obtain an adhesive composition, wherein the desalting agent is selected from the group consisting of an alkali metal hydroxide, an alkaline-earth metal hydroxide, and an alkaline-earth metal salt;
and a step of shaping the adhesive composition to obtain the adhesive layer, wherein such adhesive layer does not contain a rubber adhesive.

### Advantageous Effects of Invention

According to the method for manufacturing a patch related to the present invention, the amounts of degradation products produced of asenapine in the manufacturing of the patch can be reduced.

### Brief Description of Drawings

Figure 1 is a graph showing the amounts of degradation products produced of asenapine.
Figure 2 is a graph showing the amounts of degradation products produced of asenapine.
Figure 3 is a graph showing the amounts of degradation products produced of asenapine.

### Description of Embodiments

In the specification, the term "(meth)acrylic acid" refers to either one or both of acrylic acid and methacrylic acid, and similar expressions are defined in the same manner.

The present invention will be described in detail below with showing embodiments of the present invention. The invention is defined by the claims. Any embodiment not falling within the scope of the claims is only disclosed for illustrative purposes.

One embodiment of the present invention is a method for manufacturing a patch comprising a backing and an adhesive layer laminated on the backing, wherein the method comprises: a step of mixing an acid addition salt of asenapine, a (meth)acrylic acid ester (co)polymer and a desalting agent to obtain an adhesive composition, wherein the desalting agent is selected from the group consisting of an alkali metal hydroxide, an alkaline-earth metal hydroxide, and an alkaline-earth metal salt; and a step of shaping the adhesive composition to obtain the adhesive layer, wherein such adhesive layer does not contain a rubber adhesive.

The backing may be any backing which can maintain the form of the patch, in particular, the adhesive layer. Examples of the material of the backing include polyethylene, polypropylene, polybutadiene, an ethylene-vinyl chloride copolymer, polyvinyl chloride, polyamide such as nylon, polyester, a cellulose derivative, and a synthetic resin such as polyurethane. Examples of the shape of the backing include a film, a sheet, a sheet-like porous body, sheet-like foam, fabric such as woven fabric, knitted fabric and nonwoven fabric, and laminated products thereof. It is preferable that the thickness of the backing is normally about 2 to 3000 µm.

The adhesive layer is formed from an adhesive composition obtained by mixing an acid addition salt of asenapine, a (meth)acrylic acid ester (co)polymer and a desalting agent as claimed. The adhesive layer does not contain a rubber adhesive. Examples of the rubber adhesive include natural rubber, polyisobutylene, an alkyl vinyl ether (co)polymer, polyisoprene, polybutadiene, a styrene -butadiene copolymer, a styrene-isoprene copolymer, a styrene-isoprene-styrene block copolymer, and silicone rubber.

The thickness of the adhesive layer may be 30 to 300 µm. If the thickness of the adhesive layer is over 300 µm, the patch tends to easily fall off, for example when clothes are put on or off.

Asenapine is a compound which is also referred to as (3aRS,12bRS)-5-chloro -2-methyl-2,3,3a,12b-tetrahydro-1H-dibenzo[2,3:6,7]oxepino[4,5-c]pyrrole and is represented by the following chemical formula. Asenapine has a plurality of optical isomers and may be any optical isomer, and may be a mixture of optical isomers such as a racemate. The acid added to asenapine is pharmaceutically acceptable. The acid addition salt of asenapine may be anhydride or hydrate.

Examples of the acid include hydrochloric acid, hydrobromic acid, hydroiodic acid, phosphoric acid, acetic acid, propionic acid, glycolic acid, maleic acid, malonic acid, succinic acid, tartaric acid, citric acid, ascorbic acid, salicylic acid and benzoic acid. The acid addition salt of asenapine may be anhydride or hydrate. For example, asenapine maleate is commercially available as a therapeutic agent of schizophrenia.

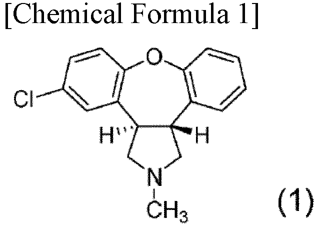

The desalting agent can convert an acid addition salt of asenapine into an asenapine free base by salt exchange reaction with an acid addition salt of asenapine. Namely, a desalting agent refers to a component which converts an acid addition salt of asenapine into an asenapine free base. The desalting agent of the invention is selected from the group consisting of an alkali metal hydroxide, an alkaline-earth metal hydroxide, and an alkaline-earth metal salt. Examples of the alkali metal hydroxide include lithium hydroxide, sodium hydroxide and potassium hydroxide. The desalting agent can be selected in consideration of pKa of the acid added to asenapine. When the desalting agent is sodium hydroxide, the degradation of agents is less in the manufacturing of the patch.

The (meth)acrylic acid ester (co)polymer is a component which gives adhesiveness to the adhesive layer, and examples of the (meth)acrylic acid ester (co)polymer include a (co)polymer of one or two or more types of (meth)acrylic acid alkyl ester. Examples of the (meth)acrylic acid alkyl ester include butyl (meth)acrylate, isobutyl (meth)acrylate, hexyl (meth)acrylate, octyl (meth)acrylate, 2-ethylhexyl (meth)acrylate and decyl (meth)acrylate.

The (meth)acrylic acid ester (co)polymer may be a copolymer formed from (meth)acrylic acid alkyl ester (a main monomer) and a comonomer. The comonomer may be any component which can copolymerize with (meth)acrylic acid alkyl ester. Examples of the comonomer include (meth)acrylic acid hydroxyalkyl ester, ethylene, propylene, styrene, vinyl acetate, N-vinylpyrrolidone and amide (meth)acrylate. One of these comonomers may be used alone or two or more may be used in combination.

Specific examples of the (meth)acrylic acid ester (co)polymer include DURO-TAK(R) 87-900A, DURO-TAK 87-2510, DURO-TAK 87-4287, DURO-TAK 87-2194 (trade name, manufactured by Henkel). DURO-TAK 87-2510 and DURO-TAK 87-4287 have a hydroxy group in its chemical structure and DURO-TAK 87-2194 has a carboxy group in its chemical structure.

The content of the (meth)acrylic acid ester (co)polymer is 50 to 98% by mass relative to the total mass of the adhesive layer and it is preferable that the content of the (meth)acrylic acid ester (co)polymer be 70 to 96% by mass. The content of the (meth)acrylic acid ester (co)polymer is calculated based on the mass of the solid content.

The adhesive layer may further contain other additives. Examples of the other additives include a tackifier resin, a plasticizer, an absorption promoting agent, a solubilizer, a stabilizing agent, a filler and a flavor.

The tackifier resin is a component which adjusts the adhesiveness of the adhesive layer. Examples of the tackifier resin include an alicyclic saturated hydrocarbon resin; rosin and rosin derivatives such as glycerine ester of rosin, hydrogenated rosin, glycerine ester of hydrogenated rosin, pentaerythritol ester of rosin and maleated rosin; a terpene tackifier resin; a petroleum tackifier resin. One of the tackifier resins may be used alone or two or more may be used in combination.

The absorption promoting agent is a component which adjusts the skin permeability of asenapine or an acid addition salt of asenapine. The absorption promoting agent is a compound whose absorption promoting action to skin is conventionally recognized, and examples of the absorption promoting agent include an aliphatic alcohol having 6 to 20 carbon atoms, an aliphatic ether having 6 to 20 carbon atoms, a fatty acid having 6 to 20 carbon atoms, a fatty ester having 6 to 20 carbon atoms, a fatty acid amide having 6 to 20 carbon atoms, glycerine, glycerine fatty esters, propylene glycols, propylene glycol fatty esters, polyethylene glycol and polyethylene glycol fatty esters, an aromatic organic acid, an aromatic alcohol, an aromatic organic acid ester, an aromatic organic ether (the above compounds may be saturated or unsaturated, and may be linear or branched, and may comprise a cyclic structure), lactic acid esters, acetic acid esters, a monoterpenes compound, a sesquiterpene compound, 1-dodecylazacycloheptane-2-one (Azone (trade name)) and derivatives thereof, sorbitan fatty esters (a series of Span(R)), a series of polysorbate (a series of Tween(R)), polyoxyethylene hydrogenated castor oils, polyoxyethylene alkylethers, sucrose fatty acid esters and a plant oil. Specific examples of the absorption promoting agent include caprylic acid, capric acid, caproic acid, lauric acid, myristic acid, palmitic acid, stearic acid, isostearic acid, oleic acid, linoleic acid, linolenic acid, lauryl alcohol, myristyl alcohol, oleyl alcohol, isostearyl alcohol, cetyl alcohol, methyl laurate, hexyl laurate, diethanolamide laurate, isopropyl myristate, myristyl myristate, octyldodecyl myristate, cetyl palmitate, isopropyl palmitate, salicylic acid, methyl salicylate, ethylene glycol salicylate, cinnamic acid, methyl cinnamate, cresol, cetyl lactate, lauryl lactate, ethyl acetate, propyl acetate, geraniol, thymol, eugenol, terpineol, 1-menthol, borneoroll, d-limonene, isoeugenol, isobomeol, nerol, dl-camphor, glycerin monocaprylate, glycerin monocaprate, glycerine monolaurate, glycerine monooleate, sorbitan monolaurate, sucrose monolaurate, polysorbate 20, propylene glycol, propylene glycol monolaurate, polyethylene glycol monolaurate, polyethylene glycol monostearate, polyoxyethylene lauryl ether, NIKKOL(R) HCO-60, pyrrothiodecane(R), olive oil and sorbitan monooleate. It is preferable that the absorption promoting agent be propylene glycol monolaurate, isopropyl palmitate, and it is more preferable that the absorption promoting agent be isopropyl palmitate, in that they remarkably improve the skin permeability of asenapine. One of the absorption promoting agents may be used alone or two or more may be used in combination.

When the adhesive layer contains the absorption promoting agent, it is preferable that the content of the absorption promoting agent be 2 to 40% by mass relative to the total mass of the adhesive layer. When the content of the absorption promoting agent is 2% by mass or more, the skin permeability of asenapine is increased and the asenapine-containing patch tends to exhibit enough pharmacological action. Further when the content of the absorption promoting agent is 40% by mass or less, skin irritancy tends not to be exhibited.

The solubilizer is a component which promotes the dissolution of asenapine and an acid addition salt thereof in the adhesive composition.

The stabilizing agent may be any stabilizing agent which can inhibit the production of free-radicals produced by the action of light such as UV rays, heat or active chemical species and the progression of the chain reaction of free-radicals. Inclusion of the stabilizing agent can further improve the stability of asenapine in the manufacturing of the patch. Examples of the stabilizing agent include tocopherol and ester derivatives thereof, ascorbic acid and ester derivatives thereof, dibutylhydroxytoluene, butylhydroxyanisole, and 2-mercaptobenzimidazole. One of the stabilizing agents may be used alone or two or more may be used in combination. It is preferable that the stabilizing agent be dibutylhydroxytoluene in that physical properties (formability and appearance) for a patch become appropriate and that asenapine is more stabilized.

When the adhesive layer contains the stabilizing agent, it is preferable that the content of the stabilizing agent be 0.1 to 3% by mass relative to the total mass of the adhesive layer. When the content of the stabilizing agent is 0.1 to 3% by mass, the stability of the components in the patch tends to be excellent.

The patch may further comprise a release liner. The release liner is laminated on the side opposite to the backing on the adhesive layer. When the patch comprises a release liner, the deposition of dirt onto the adhesive layer tents to be reduced during storage.

The raw material of the release liner is not specifically limited, and liners generally known to those in the art may be used. Examples of the material of the release liner include polyester such as polyethylene terephthalate and polyethylene naphthalate; polyolefin such as polyethylene and polypropylene; films of polyvinyl chloride or polyvinylidene chloride; laminated films of fine quality paper and polyolefin; films of Nylon (trade name) or aluminium. It is preferable that the material of the release liner be polypropylene or polyethylene terephthalate.

### <Method of manufacturing a patch>

The present invention is directed to a method for manufacturing a patch, wherein the method is as follows. First, an acid addition salt of asenapine, a (meth)acrylic acid ester (co)polymer and a desalting agent as claimed are mixed, and a solvent and other additives are added there as needed and the mixture is mixed to obtain a homogeneous adhesive composition. Then, the obtained adhesive composition is applied onto one side of a backing in a given thickness and then is heated as needed to dry and remove the solvent, and the obtained sheet is cut into a desired size to obtain the patch. Heating conditions can be selected as appropriate depending on the solvent and it is preferable that temperature conditions be 60 to 120°C, and heating time is, for example, 2 to 30 minutes.

When a patch comprising a release liner is manufactured, an adhesive composition is applied to a backing, and then the solvent is dried and removed, and then the release liner can be laminated. Also, when a patch comprising a release liner is manufactured, the patch can be obtained by applying an adhesive composition onto one side of the release liner in a given thickness, and then heating the composition as needed to dry and remove the solvent, and layering a backing, and cutting the obtained sheet into a desired size.

The adhesive composition used in the above manufacturing methods contains an acid addition salt of asenapine, a (meth)acrylic acid ester (co)polymer and a desalting agent as claimed, and further contains a solvent and other additives as needed.

The solvent may be a solvent which does not react with other components contained in the adhesive composition, and is added to adjust the viscosity of the adhesive composition. Examples of the solvent include toluene, ethanol, methanol and ethyl acetate. One of the solvent may be used alone or two or more may be used in combination. The content of the solvent may be adjusted in consideration of the viscosity of the adhesive composition.

It is preferable that the content of an acid addition salt of asenapine in the adhesive composition be 0.5 to 50% by mass relative to the total mass of the adhesive composition, and it is more preferable that the content of an acid addition salt of asenapine in the adhesive composition be 4.2 to 21% by mass.

It is preferable that the content of the (meth)acrylic acid ester (co)polymer in the adhesive composition be 50 to 98% by mass relative to the total mass of the adhesive composition, and it is more preferable that the content of the (meth)acrylic acid ester (co)polymer in the adhesive composition be 70 to 96% by mass. When the content of the (meth)acrylic acid ester (co)polymer is 50% by mass or more, cohesion is not decreased and the adhesion tends not to be decreased.

It is preferable that the content of the desalting agent in the adhesive composition be 0.05 to 20% by mass relative to the total mass of the adhesive composition, and it is more preferable that the content of the desalting agent in the adhesive composition be 0.1 to 10% by mass.

### Examples

The present invention will be described in more detail below with showing Examples and Comparative Examples. DURO-TAK 87-2516, DURO-TAK 87-4287, DURO-TAK 87-2194, DURO-TAK 87-900A and DURO-TAK 87-2510 were calculated based on the mass of the solid content.

### (1) Manufacturing of patches

The components described in Table 1 were each weighed out and solvents were added there as needed and the mixtures were mixed to obtain adhesive compositions. The obtained adhesive compositions were applied to polyester release liners and the solvents were dried and removed to form adhesive layers. Polyester films (backings) were laminated on the obtained adhesive layers and the obtained sheets were cut as appropriate to obtain desired patches. The figures in Table 1 refer to figures in % by mass.

**[Table 1]**

| | Comparative Example 1 | Comparative Example 2 | Comparative Example 3 | Comparative Example 4 |
|---|---|---|---|---|
| Asenapine | 15 | 15 | 15 | 15 |
| Asenapine maleate | - | - | - | - |
| DURO-TAK 87-2516 | 85 | 76.6 | 74.9 | 78.6 |
| Disodium maleate | - | 8.4 | - | - |
| Monosodium maleate trihydrate | - | - | 10.1 | - |
| Monolithium maleate | - | - | - | 6.4 |
| Dipotassium maleate | - | - | - | - |
| Sodium hydroxide | - | - | - | - |
| Lithium hydroxide | - | - | - | - |
| Total | 100 | 100 | 100 | 100 |

| | Comparative Example 5 | Example 1 | Example 2 | |
|---|---|---|---|---|
| Asenapine | 15 | - | - | |
| Asenapine maleate | - | 21.1 | 21.1 | |
| DURO-TAK 87-2516 | 74.9 | 74.7 | 76.4 | |
| Disodium maleate | - | - | - | |
| Monosodium maleate trihydrate | - | - | - | |
| Monolithium maleate | - | - | - | |
| Dipotassium maleate | 10.1 | - | - | |
| Sodium hydroxide | - | 4.2 | - | |
| Lithium hydroxide | - | - | 2.5 | |
| Total | 100 | 100 | 100 | |

### (2) Measurement of amounts of degradation products produced

The adhesive layers of the obtained patches were immersed in 10 mL of tetrahydrofuran (a grade for high performance liquid chromatography) and were extracted, and 40 mL of the solvent described below as a mobile phase was added there and the mixtures were filtered, and then the amounts of degradation products produced of asenapine were measured by high performance liquid chromatography under the following analysis conditions. The amounts of degradation products produced of asenapine were shown as the values of the area under the curve of the peaks corresponding to the degradation products of asenapine relative to the area under the curve of the peak corresponding to asenapine in the obtained chromatograms. The amounts of degradation products produced of asenapine were calculated taking the area under the curve of the peak corresponding to asenapine as 100.

### <Analysis conditions>

Column: TSK-gel ODS-80Ts
Mobile phase: methanol: 0.1% phosphoric acid (added with 10 mM
sodium dodecyl sulfate) = 75:25
Measurement wavelength: 210 nm
Flow rate: 1.0 mL/min
Column temperature: 40°C
Sample injection volume: 10 µL

The peak having the retention time shorter than the retention time of asenapine is referred to as "Degradation product 1 of asenapine" and the peak having retention time longer than the retention time of asenapine is referred to as "Degradation product 2 of asenapine".

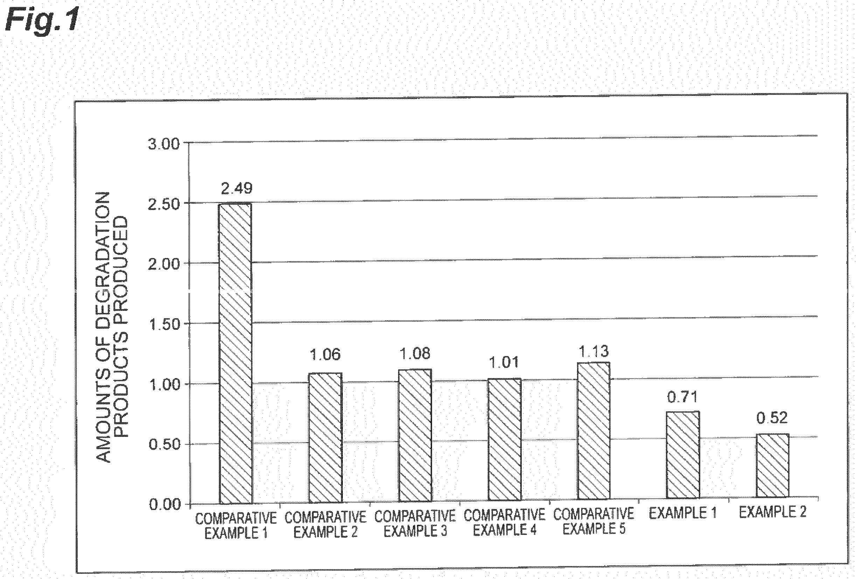

The results are shown in Table 2 and Figure 1. The degradation products of asenapine of the patches of Examples 1 and 2 were remarkably reduced compared to those of the patches of Comparative Example 1 to 5.

**[Table 2]**

| | Comparative Example 1 | Comparative Example 2 | Comparative Example 3 | Comparative Example 4 |
|---|---|---|---|---|
| Asenapine | 100 | 100 | 100 | 100 |
| Degradation product 1 | 2.09 | 0.65 | 0.72 | 0.65 |
| Degradation product 2 | 0.40 | 0.41 | 0.36 | 0.36 |
| Total of Degradation products | 2.49 | 1.06 | 1.08 | 1.01 |

| | Comparative Example 5 | Example 1 | Example 2 | |
|---|---|---|---|---|
| Asenapine | 100 | 100 | 100 | |
| Degradation product 1 | 0.78 | 0.51 | 0.34 | |
| Degradation product 2 | 0.35 | 0.20 | 0.18 | |
| Total of Degradation products | 1.13 | 0.71 | 0.52 | |

### (3) Manufacturing of patches

The components described in Table 3 were each weighed out and solvents were added there as needed and the mixtures were mixed to obtain adhesive compositions. The obtained adhesive compositions were applied to polyester release liners, and the solvent was dried and removed to form adhesive layers having a thickness of about 100 µm. Polyester films (backings) were laminated on the obtained adhesive layers and the obtained sheets were cut as appropriate to obtain desired patches. The figures in Table 3 refer to figures in % by mass.

**[Table 3]**

| | Comparative Example 6 | Comparative Example 7 | Comparative Example 8 | Example 3 |
|---|---|---|---|---|
| Asenapine | 15 | 15 | 15 | - |
| Asenapine maleate | - | - | - | 21.1 |
| DURO-TAK 87-900A | 85 | 76.6 | 74.9 | 74.7 |
| DURO-TAK 87-2510 | - | - | - | - |
| Disodium maleate | - | 8.4 | - | - |
| Monosodium maleate trihydrate | - | - | 10.1 | - |
| Sodium hydroxide | - | - | - | 4.2 |
| Total | 100 | 100 | 100 | 100 |

| | Comparative Example 9 | Comparative Example 10 | Comparative Example 11 | Example 4 |
|---|---|---|---|---|
| Asenapine | 15 | 15 | 15 | - |
| Asenapine maleate | - | - | - | 21.1 |
| DURO-TAK 87-900A | - | - | - | - |
| DURO-TAK 87-2510 | 85 | 76.6 | 74.9 | 74.7 |
| Disodium maleate | - | 8.4 | - | - |
| Monosodium maleate trihydrate | - | - | 10.1 | - |
| Sodium hydroxide | - | - | - | 4.2 |
| Total | 100 | 100 | 100 | 100 |

### (4) Measurement of amounts of degradation products produced

The adhesive layers of the obtained patches were immersed in 10 mL of tetrahydrofuran (a grade for high performance liquid chromatography) and were extracted, and 40 mL of the solvent described above as a mobile phase was added there and the mixtures were filtered, and then the amounts of degradation products produced of asenapine were measured by high performance liquid chromatography under the above mentioned analysis conditions.

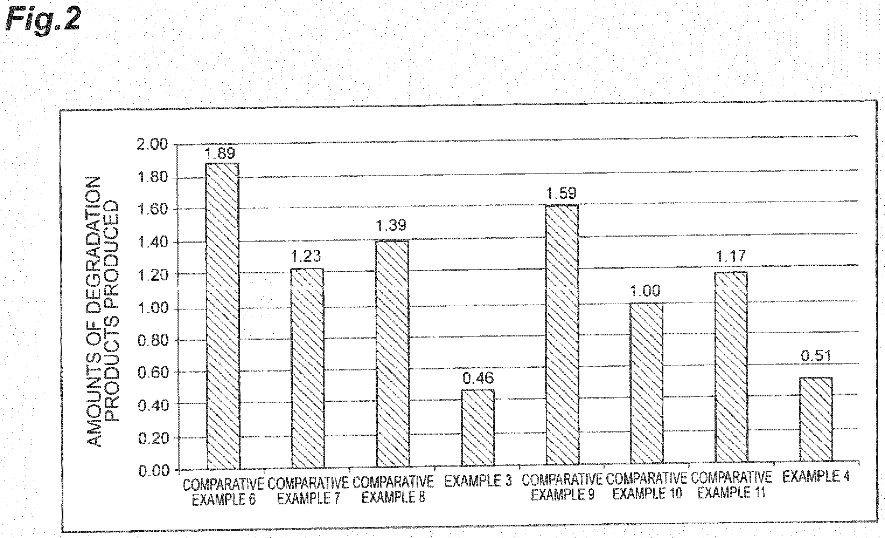

The results are shown in Table 4 and Figure 2. The degradation products of asenapine of the patches of Examples 3 and 4 were remarkably reduced compared to those of the patches of Comparative Example 6 to 11.

**[Table 4]**

| | Comparative Example 6 | Comparative Example 7 | Comparative Example 8 | Example 3 |
|---|---|---|---|---|
| Asenapine | 100 | 100 | 100 | 100 |
| Degradation product 1 | 1.77 | 1.14 | 1.3 | 0.43 |
| Degradation product 2 | 0.12 | 0.09 | 0.09 | 0.03 |
| Total of Degradation products | 1.89 | 1.23 | 1.39 | 0.46 |

| | Comparative Example 9 | Comparative Example 10 | Comparative Example 11 | Example 4 |
|---|---|---|---|---|
| Asenapine | 100 | 100 | 100 | 100 |
| Degradation product 1 | 1.42 | 0.85 | 1.02 | 0.26 |
| Degradation product 2 | 0.17 | 0.15 | 0.15 | 0.25 |
| Total of Degradation products | 1.59 | 1.00 | 1.17 | 0.51 |

### (5) Manufacturing of patches

The components described in Table 5 were each weighed out and solvents were added there as needed and the mixtures were mixed to obtain adhesive compositions. The obtained adhesive compositions were applied to polyester release liners and the solvents were dried and removed to form adhesive layers. Polyester films (backings) were laminated on the obtained adhesive layers and the obtained sheets were cut as appropriate to obtain desired patches. The figures in Table 5 refer to figures in % by mass.

**[Table 5]**

| | Comparative Example 12 | Comparative Example 13 | Comparative Example 14 | Example 5 |
|---|---|---|---|---|
| Asenapine | 15 | 15 | 15 | - |
| Asenapine maleate | - | - | - | 21.1 |
| DURO-TAK 87-4287 | 85 | 76.6 | 74.9 | 74.7 |
| DURO-TAK 87-2194 | - | - | - | - |
| Disodium maleate | - | 8.4 | - | - |
| Monosodium maleate trihydrate | - | - | 10.1 | - |
| Sodium hydroxide | - | - | - | 4.2 |
| Total | 100 | 100 | 100 | 100 |

| | Comparative Example 15 | Comparative Example 16 | Comparative Example 17 | Example 6 |
|---|---|---|---|---|
| Asenapine | 15 | 15 | 15 | - |
| Asenapine maleate | - | - | - | 21.1 |
| DURO-TAK 87-4287 | - | - | - | - |
| DURO-TAK 87-2194 | 85 | 76.6 | 74.9 | 74.7 |
| Disodium maleate | - | 8.4 | - | - |
| Monosodium maleate trihydrate | - | - | 10.1 | - |
| Sodium hydroxide | - | - | - | 4.2 |
| Total | 100 | 100 | 100 | 100 |

### (6) Measurement of amounts of degradation products produced

The adhesive layers of the obtained patches were immersed in 10 mL of tetrahydrofuran (a grade for high performance liquid chromatography) and were extracted, and 40 mL of the solvent described above as a mobile phase was added there and the mixtures were filtered, and then the amounts of degradation products produced of asenapine were measured by high performance liquid chromatography under the above mentioned analysis conditions.

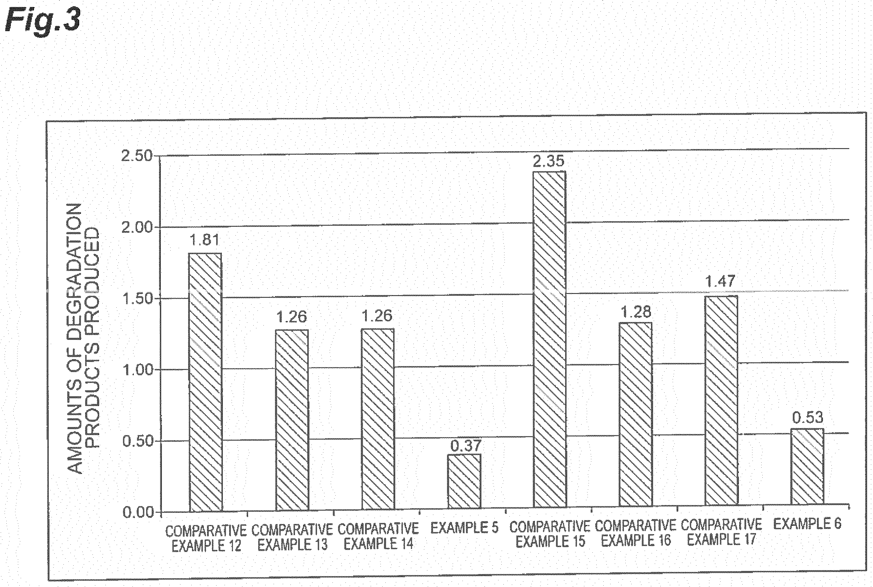

The results are shown in Table 6 and Figure 3. The degradation products of asenapine of the patches of Examples 5 and 6 were remarkably reduced compared to those of the patches of Comparative Example 12 to 17.

**[Table 6]**

| | Comparative Example 12 | Comparative Example 13 | Comparative Example 14 | Example 5 |
|---|---|---|---|---|
| Asenapine | 100 | 100 | 100 | 100 |
| Degradation product 1 | 1.6 | 1.21 | 1.23 | 0.34 |
| Degradation product 2 | 0.21 | 0.05 | 0.03 | 0.03 |
| Total of Degradation products | 1.81 | 1.26 | 1.26 | 0.37 |

| | Comparative Example 15 | Comparative Example 16 | Comparative Example 17 | Example 6 |
|---|---|---|---|---|
| Asenapine | 100 | 100 | 100 | 100 |
| Degradation product 1 | 1.98 | 0.96 | 1.15 | 0.25 |
| Degradation product 2 | 0.37 | 0.32 | 0.32 | 0.28 |
| Total of Degradation products | 2.35 | 1.28 | 1.47 | 0.53 |

### (7) Investigation of desalting agents

A patch (Example 7) was prepared by using an equimolar amount of potassium hydroxide instead of sodium hydroxide in the patch of Example 1. The amounts of degradation products produced of asenapine in the patch of Example 7 were measured and it was found that the amounts of degradation products produced were decreased. Further, a patch was prepared by using an equimolar amount of sodium carbonate (Example 8), by using sodium bicarbonate (Example 9), by using potassium carbonate (Example 10) and by using potassium bicarbonate (Example 11) instead of sodium hydroxide in the patch of Example 1. Examples 8-11 are not according to the invention.

### (8) Manufacturing of patches

The components described in Table 7 were each weighed out and solvents were added there as needed and the mixtures were mixed to obtain adhesive compositions. The obtained adhesive compositions were applied to polyester release liners and the solvents were dried and removed to form adhesive layers having a thickness of about 100 µm. Polyester films (backings) were laminated on the obtained adhesive layers and the obtained sheets were cut as appropriate to obtain desired patches. The figures in Table 7 refer to figures in % by mass. Examples 12-14 are not according to the invention.

**[Table 7]**

| | Example 12 | Example 13 | Example 14 | Comparative Example 18 |
|---|---|---|---|---|
| Asenapine | - | - | - | 8.5 |
| Asenapine maleate | 12 | 12 | 12 | - |
| DURO-TAK 87-2516 | 79.09 | 82.98 | 68.86 | 91.5 |
| Triethanolamine | 8.91 | - | - | - |
| Sodium bicarbonate | - | 5.02 | - | - |
| Sodium oleate | - | - | 19.14 | - |
| Total | 100 | 100 | 100 | 100 |

### (9) Measurement of amounts of degradation products produced

The results are shown in Table 8. The degradation products of asenapine of the patches of Examples 12 to 14 were remarkably reduced compared to those of the patch of Comparative Example 18. "N.D." in Table 8 means that the value is below the detection limit.

**[Table 8]**

| | Example 12 | Example 13 | Example 14 | Comparative Example 18 |
|---|---|---|---|---|
| Degradation product 1 | N.D. | N.D. | 0.16 | 1.22 |
| Degradation product 2 | 0.10 | 0.12 | 0.10 | 0.31 |
| Total of Degradation products | 0.10 | 0.12 | 0.26 | 1.53 |

### (10) Manufacturing of patches

The components described in Table 9 were each weighed out and solvents were added there as needed and the mixtures were mixed to obtain adhesive compositions. The obtained adhesive compositions were applied to polyester release liners and the solvents were dried and removed to form adhesive layers having a thickness of about 100 µm. Polyester films (backings) were laminated on the obtained adhesive layers and the obtained sheets were cut as appropriate to obtain desired patches. The figures in Table 9 refer to figures in % by mass.

**[Table 9]**

| | Example 15 | Example 16 | Example 17 |
|---|---|---|---|
| Asenapine maleate | 0.50 | 4.22 | 8.44 |
| DURO-TAK 87-2516 | 99.40 | 94.94 | 89.88 |
| Sodium hydroxide | 0.10 | 0.84 | 1.68 |
| Total | 100 | 100 | 100 |

| | Example 18 | Example 19 | Example 20 |
|---|---|---|---|
| Asenapine maleate | 21 | 35 | 50 |
| DURO-TAK 87-2516 | 74.82 | 58.03 | 40.05 |
| Sodium hydroxide | 4.18 | 6.97 | 9.95 |
| Total | 100 | 100 | 100 |

### (11) Measurement of amounts of degradation products produced

The result is shown in Table 10. The degradation products of asenapine of the patches of Examples 15 to 20 were remarkably small.

**[Table 10]**

| | Example 15 | Example 16 | Example 17 |
|---|---|---|---|
| Degradation product 1 | 0.34 | 0.27 | 0.45 |
| Degradation product 2 | 0.08 | 0.23 | 0.24 |
| Total of Degradation products | 0.42 | 0.50 | 0.69 |

| | Example 18 | Example 19 | Example 20 |
|---|---|---|---|
| Degradation product 1 | 0.51 | 0.35 | 0.06 |
| Degradation product 2 | 0.20 | 0.12 | 0.00 |
| Total of Degradation products | 0.71 | 0.47 | 0.06 |

## Claims

1. A method for manufacturing a patch comprising a backing and an adhesive layer laminated on the backing, comprising:
a step of mixing an acid addition salt of asenapine, a (meth)acrylic acid ester (co)polymer and a desalting agent to obtain an adhesive composition, wherein the desalting agent is selected from the group consisting of an alkali metal hydroxide, an alkaline-earth metal hydroxide, and an alkaline-earth metal salt; and
a step of shaping the adhesive composition to obtain the adhesive layer, wherein such adhesive layer does not contain a rubber adhesive.

2. The method according to claim 1, wherein the acid in the acid addition salt of asenapine is selected from the group consisting of hydrochloric acid, hydrobromic acid, hydriodic acid, phosphoric acid, acetic acid, propionic acid, glycolic acid, maleic acid, malonic acid, succinic acid, tartaric acid, citric acid, ascorbic acid, salicylic acid and benzoic acid.

3. A method for inhibiting the degradation of asenapine in a manufacturing process of a patch comprising an adhesive layer comprising asenapine, comprising:
a step of mixing an acid addition salt of asenapine, a (meth)acrylic acid ester (co)polymer and a desalting agent to obtain an adhesive composition, wherein the desalting agent is selected from the group consisting of an alkali metal hydroxide, an alkaline-earth metal hydroxide, and an alkaline-earth metal salt; and
a step of shaping the adhesive composition to obtain the adhesive layer, wherein such adhesive layer does not contain a rubber adhesive.

## Patentansprüche

1. Verfahren zur Herstellung eines Pflasters, umfassend eine Unterlage und eine auf die Unterlage laminierte Klebstoffschicht, umfassend:
einen Schritt des Mischens eines Säureadditionssalzes von Asenapin, eines (Meth)acrylsäureester-(Co)polymers und eines Entsalzungsmittels, um eine Klebstoffzusammensetzung zu erhalten, wobei das Entsalzungsmittel ausgewählt ist aus der Gruppe bestehend aus einem Alkalimetallhydroxid, einem Erdalkalimetallhydroxid und einem Erdalkalimetallsalz; und
einen Schritt des Formens der Klebstoffzusammensetzung, um die Klebstoffschicht zu erhalten, wobei diese Klebstoffschicht keinen Kautschukklebstoff enthält.

2. Verfahren nach Anspruch 1, wobei die Säure in dem Säureadditionssalz von Asenapin ausgewählt ist aus der Gruppe ausgewählt bestehend aus Salzsäure, Bromwasserstoffsäure, lodwasserstoffsäure, Phosphorsäure, Essigsäure, Propionsäure, Glykolsäure, Maleinsäure, Malonsäure, Bernsteinsäure, Weinsäure, Zitronensäure, Ascorbinsäure, Salicylsäure und Benzoesäure.

3. Verfahren zur Hemmung des Abbaus von Asenapin in einem Herstellungsprozess eines Pflasters umfassend eine Klebstoffschicht umfassend Asenapin, umfassend:
einen Schritt des Mischens eines Säureadditionssalzes von Asenapin, eines (Meth)acrylsäureester-(Co)polymers und eines Entsalzungsmittels, um eine Klebstoffzusammensetzung zu erhalten, wobei das Entsalzungsmittel ausgewählt ist aus der Gruppe bestehend aus einem Alkalimetallhydroxid, einem Erdalkalimetallhydroxid und einem Erdalkalimetallsalz; und
einen Schritt des Formens der Klebstoffzusammensetzung, um die Klebstoffschicht zu erhalten, wobei diese Klebstoffschicht keinen Kautschukklebstoff enthält.

## Revendications

1. Procédé de fabrication d'un timbre comprenant un support et une couche adhésive laminée sur le support, comprenant:
une étape consistant à mélanger un sel d'addition d'acide d'asénapine, un (co)polymère d'ester d'acide (méth)acrylique et un agent de dessalage pour obtenir une composition adhésive, dans laquelle l'agent de dessalage est choisi dans le groupe constitué par un hydroxyde de métal alcalin, un hydroxyde de métal alcalino-terreux et un sel de métal alcalino-terreux;
et
une étape consistant à mettre en forme la composition adhésive pour obtenir la couche adhésive, dans laquelle ladite couche adhésive ne contient pas d'adhésif à base de caoutchouc.

2. Procédé selon la revendication 1, dans lequel l'acide dans le sel d'addition d'acide de l'asénapine est choisi dans le groupe constitué par l'acide chlorhydrique, l'acide bromhydrique, l'acide iodhydrique, l'acide phosphorique, l'acide acétique, l'acide propionique, l'acide glycolique, l'acide maléique, l'acide malonique, l'acide succinique, l'acide tartrique, l'acide citrique, l'acide ascorbique, l'acide salicylique et l'acide benzoïque.

3. Procédé pour inhiber la dégradation de l'asénapine dans un processus de fabrication d'un timbre comprenant une couche adhésive comprenant de l'asénapine, comprenant:
une étape consistant à mélanger un sel d'addition d'acide de l'asénapine, un (co)polymère d'ester d'acide (méth)acrylique et un agent de dessalement pour obtenir une composition adhésive, dans laquelle l'agent de dessalement est choisi dans le groupe constitué par un hydroxyde de métal alcalin, un hydroxyde de métal alcalino-terreux et un sel de métal alcalino-terreux;
et
une étape consistant à mettre en forme la composition adhésive pour obtenir la couche adhésive, dans laquelle ladite couche adhésive ne contient pas d'adhésif à base de caoutchouc.
